Europäisches Patentamt

European Patent Office

Office européen des brevets

(19)

(11) Veröffentlichungsnummer: **0 203 506**
**A1**

(12) **EUROPÄISCHE PATENTANMELDUNG**

(21) Anmeldenummer: **86106873.2**

(22) Anmeldetag: **21.05.86**

(51) Int. Cl.⁴: **C 07 C 143/55**

(30) Priorität: **31.05.85 DE 3519552**

(43) Veröffentlichungstag der Anmeldung: **03.12.86**
**Patentblatt 86/49**

(84) Benannte Vertragsstaaten: **CH DE FR GB IT LI**

(71) Anmelder: **BAYER AG, Konzernverwaltung RP**
**Patentabteilung, D-5090 Leverkusen 1 Bayerwerk (DE)**

(72) Erfinder: **Skipka, Guido, Dr., Stockemer Strasse 91,**
**D-5190 Stolberg (DE)**
Erfinder: **Dürholz, Friedrich, Dr., Knusthöhe 40,**
**D-5630 Remscheid (DE)**

(54) Verfahren zur Herstellung von 4,4'-Dinitrostilben -2,2'-disulfonsäuresalzen.

(57) 4,4'-Dinitrostilben-2,2'- disulfonsäuresalze werden hergestellt durch Oxidation von 4-Nitrotoluol-2- sulfonsäure oder deren Salzen in wässrig-alkalischem Medium, indem man während der Reaktion Kalium-, Calcium- und/oder Magnesiumionen in dem Masse zugibt wie 4,4'-Dinitrostilben-2,2'- disulfonsäure gebildet wird, wobei zu jedem Zeitpunkt der Reaktion die Menge der zugegebenen Kalium-, Calcium- und/oder Magnesiumionen 10 bis 150 Mol-%, bezogen auf die jeweils im Reaktionsgemisch vorhandene Menge an 4,4'-Dinitrostilben-2,2'- disulfonsäure beträgt, und das ausgefallene Salz der 4,4'-Dinitrostilben-2,2'- disulfonsäure abtrennt.

EP 0 203 506 A1

BAYER AKTIENGESELLSCHAFT          5090 Leverkusen, Bayerwerk

Konzernverwaltung RP

Patentabteilung                   Bg/by-c


**Verfahren zur Herstellung von 4,4'-Dinitrostilben-2,2'-disulfonsäuresalzen**

Die Erfindung betrifft ein Verfahren zur Herstellung von 4,4'-Dinitrostilben-2,2'-disulfonsäuresalzen durch Oxidation von 4-Nitrotoluol-2-sulfonsäure oder deren Salzen in wäßrig-alkalischem Medium.

Die Herstellung von 4,4'-Dinitrostilben-2,2'-disulfonsäure bzw. deren Salzen (nachfolgend Dinitrostilbensäure genannt) ist seit langem bekannt und besteht in der oxidativen Kondensation von 2 Mol 4-Nitrotoluol-2-sulfonsäure (nachfolgend p-Nitrotoluolsulfonsäure genannt) unter wäßrig-alkalischen Bedingungen. Als Oxidationsmittel wurden Sauerstoff (Luft), meist in Gegenwart eines Katalysators, oder Natriumhypochlorit bzw. Chlor eingesetzt (vgl. z.B. F. Bender und G. Schultz, Ber. 19, 3234; O. Fischer und E. Hepp, Ber. 26, 2233; DE-OS 22 58 530). Aufgrund der schlechten Ausbeuten der bekannten technischen Verfahren von nur ca. 75 % versuchte man durch Änderung der Reaktionsbedingungen die Ausbeuten an Dinitrostilbensäure zu steigern.

Le A 23 691-Ausland

So wird gemäß der SU-Patentschrift 230 136 die Natronlauge in zwei Stufen mit verschiedenen Konzentrationen
zugegeben (ca. 2 %ige Natronlauge in der ersten und ca.
4 %ige Natronlauge in der zweiten Stufe). Die Ausbeute
an Dinitrostilbensäure beträgt allerdings nur maximal
65 % der Theorie.

In der DDR-Patentschrift 204 272 wird ein Verfahren zur
Herstellung von Dinitrostilbensäure durch Oxidation von
p-Nitrotoluolsulfonsäure in wäßrig-alkalischem Medium
mittels Luftsauerstoff beschrieben, das dadurch gekennzeichnet ist, daß die Konzentration der p-Nitrotoluolsulfonsäure in der Oxidationslösung um so niedriger gehalten wird, je höher die Alkalikonzentration und/oder
die Reaktionstemperatur liegen. Die Ausbeuten an Dinitrostilbensäure werden mit 70 bis 80 % der Theorie angegeben.

Aus der polnischen Patentschrift 64 173 ist ein Verfahren bekannt, bei dem die Oxidation in einer 4-bödigen
Kolonne durchgeführt wird. Das Sauerstoff- Luftgemisch
mit einem Sauerstoffanteil von 20 bis 50 Vol-% wird im
Gegenstrom im geschlossenen Kreislauf geführt. Für die
Natronlauge wird ein Konzentrationsbereich von 3,1 bis
10 Gew.-% und für das Natriumsalz der p-Nitrotoluolsulfonsäure von 4 bis 8 Gew.-% angegeben. Die Ausbeuten
betragen in Abhängigkeit von den Konzentrationen der
Reaktanden 60 bis 85 % der Theorie.

Eine Ausbeutesteigerung auf maximal 82 % der Theorie an Dinitrostilbensäure wird in der japanischen Patentschrift 82 38 764 beschrieben. Neben dem Katalysator Mangansulfat werden noch zusätzlich Verbindungen von Molybdän, Barium, Eisen, Nickel und Kobalt bzw. deren Mischungen eingesetzt.

In der europäischen Offenlegungsschrift 26 154 ist ein Verfahren zur Herstellung von Dinitrostilbensäure und deren Salzen durch Oxidation von p-Nitrotoluolsulfonsäure in organischen Lösungsmitteln beschrieben. Nach diesem Verfahren werden Ausbeuten je nach Arbeitsweise von bis zu 95 % der Theorie erzielt. Nachteilig bei diesem Verfahren ist das Arbeiten in organischen Lösungsmitteln und die tiefen Reaktionstemperaturen.

Aus der GB-PS 21 36 430 ist ein Verfahren zur Herstellung von Dinitrostilbensäure bekannt, das dadurch gekennzeichnet ist, daß man die Oxidation der p-Nitrotoluolsulfonsäure in Gegenwart von Lithium- und Hydroxylionen gegebenenfalls unter Zusatz eines Katalysators durchführt. Die Ausbeuten bei diesem Verfahren werden mit etwa 80 bis 90 % der Theorie angegeben. Bei diesem Verfahren ist als zusätzlicher Schritt die Abtrennung von Lithiumcarbonat vor der Isolierung der Dinitrostilbensäure notwendig. Die Wiedergewinnung des Lithiumcarbonats beträgt allerdings nur 75 %. Außerdem muß das abgetrennte Lithiumcarbonat erst in Lithiumhydroxid umgewandelt werden, bevor es wieder in dem Prozeß verwendet werden kann.

Le A 23 691

Durch die vor allem aufwendige Abtrennung des Lithiumcarbonats vor der Isolierung der Dinitrostilbensäure gestaltet sich das in der GB-PS beschriebene Verfahren wenig wirtschaftlich.

Nachteilig bei den bislang bekannten Verfahren zur Herstellung von Dinitrostilbensäure sind nicht nur die alles in allem unbefriedigenden Ausbeuten sondern darüber hinaus auch die Bildung von unerwünschten Nebenprodukten. Diese belasten das Abwasser und verursachen dadurch erhebliche Kosten.

Es wurde nun ein Verfahren zur Herstellung von 4,4'-Dinitrostilben-2,2'-disulfonsäuresalzen durch Oxidation von 4-Nitrotoluol-2-sulfonsäure oder deren Salzen in wäßrig-alkalischem Medium gefunden, das dadurch gekennzeichnet ist, daß man während der Reaktion Kalium-, Calcium- und/oder Magnesiumionen in dem Maße zugibt wie 4,4'-Dinitrostilben-2,2'-disulfonsäure gebildet wird, wobei zu jedem Zeitpunkt der Reaktion die Menge der zugegebenen Kalium-, Calcium- und/oder Magnesiumionen 10 bis 150 Mol-%, bezogen auf die jeweils im Reaktionsgemisch vorhandene Menge an 4,4'-Dinitrostilben-2,2'-disulfonsäure beträgt, und die ausgefallenen Salze der 4,4'-Dinitrostilben-2,2'-disulfonsäure abtrennt.

Der Reaktionsverlauf und die Bildung der Dinitrostilbensäure im Reaktionsgemisch kann mit Hilfe der flüssigkeitschromatographischen Analyse (HPLC) gut verfolgt

Le A 23 691

werden, so daß die Dosierung der Kalium-, Calcium- und/oder Magnesiumionen optimal gesteuert werden kann. Vorteilhafterweise wird die Zugabe der Kalium-, Calcium- und/oder Magnesiumionen so gesteuert, daß zu jedem Zeitpunkt der Reaktion die Menge der zugegebenen Kalium-, Calcium- und/oder Magnesiumionen 20 bis 140 Mol-%, besonders bevorzugt 50 bis 130 Mol-%, bezogen auf die jeweils im Reaktionsgemisch vorhandene Menge an 4,4'-Dinitrostilben-2,2'-disulfonsäure beträgt.

Für das erfindungsgemäße Verfahren ist es günstig, wenn die Kalium-, Calcium- und/oder Magnesiumverbindungen in ionogener Form also z.B. als Hydroxide, Chloride und/ oder Sulfate eingesetzt werden. Es ist jedoch auch möglich schwerer lösliche Kalium-, Calcium- und/oder Magnesiumverbindungen, wie die entsprechenden Oxide, in das erfindungsgemäße Verfahren einzusetzen.

Die Oxidation der p-Nitrotoluolsulfonsäure wird erfindungsgemäß in wäßrig-alkalischem Medium durchgeführt. Zur Herstellung des wäßrig-alkalischen Mediums können verschiedene Hydroxyl-Ionen bildende Verbindungen eingesetzt werden. Eine Ausnahme bilden dabei lediglich solche hydroxylionenbildende Verbindungen, die mit der Dinitrostilbensäure schwer lösliche Salze bilden. Beispielsweise können in das erfindungsgemäße Verfahren eingesetzt werden Lithiumhydroxid, Natriumhydroxid und/ oder quartäre Ammoniumhydroxide. Im allgemeinen beträgt die Konzentration der Hydroxide im wäßrig-alkalischen Medium etwa 1 bis 15 Gew.-%, bevorzugt 2 bis 10 Gew.-%, bezogen auf das Reaktionsgemisch.

Le A 23 691

Üblicherweise beträgt die Konzentration von p-Nitrotoluolsulfonsäure im Reaktionsgemisch etwa 2 bis 30 Gew.-%, bevorzugt 4 bis 25 Gew.-%.

Das erfindungsgemäße Verfahren wird im allgemeinen bei Temperaturen von etwa 30 bis 100°C, bevorzugt bei 40 bis 80°C, durchgeführt.

Das erfindungsgemäße Verfahren kann sowohl diskontinuierlich als kontinuierlich durchgeführt werden.

Das erfindungsgemäße Verfahren wird im allgemeinen bei Normaldruck durchgeführt. Es ist jedoch auch möglich, bei vermindertem oder erhöhtem Druck zu arbeiten.

In einer vorteilhaften Ausführungsform des erfindungsgemäßen Verfahrens wird Dinitrostilbensäure im wäßrig-alkalischen Medium in Mengen von etwa 0,5 bis 30 g/l, bevorzugt 2 bis 15 g/l, vorgelegt, danach die p-Nitrotoluolsulfonsäure zugegeben und die Oxidation dann, wie beschrieben, unter weiterer Zugabe von Kalium-, Calcium- und/oder Magnesiumionen durchgeführt.

Als Oxidationsmittel kommen reiner Sauerstoff oder seine Gemische mit inerten Gasen, wie Stickstoff, insbesondere Luft, in Frage. Es ist auch möglich andere Oxidationsmittel wie Hypochlorite einzusetzen. Die Oxidationsmittel werden dabei im Überschuß bezogen auf p-Nitrotoluolsulfonsäure eingesetzt. Im allgemeinen verwendet

Le A 23 691

man einen Überschuß an Oxidationsmittel, insbesondere Sauerstoff oder Luft, von etwa 300 Gew.-%, bevorzugt 10 bis 100 Gew.-%, bezogen auf p-Nitrotoluolsulfonsäure.

Gemäß einer weiteren, vorteilhaften Ausführungsform wird die Oxidation der p-Nitrotoluolsulfonsäure zu Dinitrostilbensäure in zwei Stufen durchgeführt. Danach wird in der ersten Stufe p-Nitrotoluolsulfonsäure in einem Alkalikonzentrationsbereich von etwa 4 bis 10 Gew.-% und bei Temperaturen von etwa 45 bis 70°C mit einem Sauerstoff-Luftgemisch zu 4,4'-Dinitrodibenzyl-2,2'-disulfonsäure (im folgenden Dinitrodibenzylsäure genannt) und etwas Dinitrostilbensäure oxidiert. Dabei beträgt die Ausbeute von Dinitrodibenzylsäure und Dinitrostilbensäure in der Summe etwa 91 % neben etwa 7 % p-Nitrotoluolsulfonsäure. Ihr Bildungsverhältnis ist abhängig von der Temperatur und der Alkalikonzentration und kann analytisch leicht ermittelt werden. In dem Maße wie sich Dinitrostilbensäure bildet, werden Kalium-, Calcium- und/oder Magnesiumionen dem Reaktionsgemisch zugegeben. In der zweiten Stufe wird die Dinitrodibenzylsäure weiter zur Dinitrostilbensäure oxidiert. Dabei ist es wichtig, daß die Alkalikonzentration im wäßrig-alkalischen Medium durch Verdünnen mit Wasser auf etwa 2 bis 6 Gew.-% erniedrigt wird und die Temperatur auf etwa 50 bis 90°C gesteigert wird. In dieser Stufe erfolgt die Zugabe der restlichen Menge an Kalium-, Calcium- und/oder Magnesiumionen.

Das Reaktionsgemisch wird einige Zeit gekühlt, gegebenenfalls mit verdünnter Säure neutral gestellt und die ausgefallenen Salze der Dinitrostilbensäure in üblicher Weise abgetrennt.

Le A 23 691

Die Ausbeuten die nach dem erfindungsgemäßen Verfahren an Dinitrostilbensäure erzielt werden betragen etwa 90 bis 97 % der Theorie. Unerwünschte Nebenprodukte werden, wenn überhaupt, nur in unerheblichen Mengen gebildet.

Le A 23 691

## Beispiel 1

Zu 575 g destilliertem Wassers werden 125,3 g 9,9 gew.-%ige Natronlauge (0,31 Mol) gegeben. Nach Aufheizen der Natronlauge auf 68°C werden 117,1 g 87,8 %ige fein pulverisierte Dibenzylsäure (0,24 Mol) eingetragen. In das Reaktionsgemisch werden stündlich 60 1 Sauerstoff eingeleitet. Innerhalb von 6 Stunden werden 189 g (175 ml) 12,4 gew.-%ige Kalilauge (0,42 Mol) dem Reaktionsgemisch in der Weise zugegeben, daß man pro Stunde zwischen 50 und 15 ml Kalilauge zudosiert. Nach Zugabe der Kalilauge wird noch 1 1/2 Stunden nachgereagieren gelassen und dann 30 g Kaliumchlorid in das Reaktionsgemisch eingetragen. Durch Zugabe von 30 gew.-%iger Salzsäure wird das Reaktionsgemisch neutral gestellt und über Nacht abgekühlt. Nach Abtrennen der Mutterlauge erhält man 133 g feuchtes Produkt, was 117 g Trockenware entspricht. Die Ausbeute an Dinitrostilbensäurekaliumsalz beträgt 94,7 % der Theorie (analytisch ermittelt nach HPLC-Methode).

## Beispiel 2

Es werden 1000 g 4,5 gew.-%ige Natronlauge und 3 g 4,4'-Dinitrostilben-2,2'-disulfonsäure bei 70°C vorgelegt. Während ca. 2,5 Stunden gibt man unter Rühren 124 g fein pulverisierte 4,4'-Dinitrodibenzyl-2,2'-disulfonsäure (87,2 %ig) und 430 g 6,19 gew.-%ige wäßrige Kalilauge in kleinen Portionen (ca. 20 Portionen) bei 70°C zu. Während der gesamten Versuchsdauer wurden 50 1 Sauerstoff pro Stunde und 20 1 Luft pro Stunde in das Reaktionsgemisch eingeleitet. Nach Rühren von weiteren

Le A 23 691

2 Stunden bei 70°C fügt man noch 6 g Kalilauge (100 gew.-%ig) zu und stellt mit 210 g 30 gew.-%iger Salzsäure neutral. Durch Filtration isoliert man 145 g feuchtes Dikaliumsalz der 4,4'-Dinitrostilben-2,2'-disulfonsäure, entsprechend einer Trockenware von 124,7 g. Nach Analyse mit Hilfe der HPLC-Methode ermittelt man eine Ausbeute von 97,2 % der Theorie.

Beispiel 3

Man legt 1000 g 2,5 gew.-%ige Natronlauge bei 70°C vor; leitet 50 l Sauerstoff pro Stunde und 20 l Luft pro Stunde hinein und gibt zu der Lauge innerhalb von 2 Stunden unter Rühren 124 g fein pulverisierte 4,4'-Dinitrodibenzyl-2,2'-disulfonsäure (87,2 gew.-%ig) und 777 g 3,44 gew.-%ige Kalilauge (aufgeteilt in 20 Portionen) bei 70°C zu. Man läßt noch 2 Stunden nachreagieren, fügt 7 g Kaliumchlorid dem Reaktionsgemisch hinzu, stellt mit 30 gew.-%iger Salzsäure neutral und kühlt auf Zimmertemperatur ab. Nach Filtration erhält man 148,7 g feuchtes Dikaliumsalz der 4,4'-Dinitrostilben-2,2'-disulfonsäure entsprechend einer Trockenware von 123,5 g. Nach Analyse mit Hilfe der HPLC-Methode ermittelt man eine Ausbeute von 96,7 % der Theorie an 4,4'-Dinitrostilben-2,2'-disulfonsäure.

Beispiel 4

Die Reaktion wird in einer Kaskade kontinuierlich durchgeführt, die aus vier Sulfiergefäßen als Reaktoren mit einem Reaktionsvolumen von ca. 2,5 l Reaktionsvolumen und kontinuierlichen Überläufen besteht. Die Reaktoren sind mit Scheibenrührer und Gaseinleitungs-

Le A 23 691

rohr ausgerüstet. Die ausreagierte Reaktionsmischung wird in einem gekühlten Rührgefäß gesammelt und neutralisiert.

In die einzelnen Reaktoren werden während der Versuchsdauer 50 l pro Stunde Sauerstoff und 20 l pro Stunde Luft über ein Gaseinleitungsrohr eingeleitet.

Die Reaktoren 1 und 2 werden zum Anfahren mit 1 l 6%iger Natronlauge und die Reaktoren 3 und 4 mit 1 l 3,5%iger Natronlauge gefüllt.

Die Reaktionstemperaturen in den Reaktoren werden mit Mantelheizung auf folgende Werte eingestellt: Reaktor 1 und 2 auf 59°C, Reaktor 3 auf 72°C sowie Reaktor 4 auf 70°C.

In den Reaktor 1 werden 311 g pro Stunde p-Nitrotoluolsulfonsäure (32,8%ig) und 98,8 g pro Stunde einer Mischung aus Natronlauge und Kalilauge (93,5 g 50%ige Natronlauge und 5,3 g 50%ige Kalilauge) kontinuierlich eindosiert.

In den Reaktor 3 werden kontinuierlich 16 g 3,4%ige Kalilauge und 50 g einer 0,002%igen Mangansulfatlösung eindosiert.

Die aus dem Reaktor 4 ablaufende Reaktionssuspension wird in einem gekühlten Rührgefäß gesammelt und kontinuierlich mit Salzsäure neutralisiert.

Le A 23 691

Die während zwei Stunden gesammelte Reaktionssuspension wird zur vollständigen Ausfällung der 4,4'-Dinitrostilben-2,2'-disulfonsäure mit 30 g Kaliumchlorid versetzt und eine Stunde nachgerührt.

Die Isolierung erfolgt durch Filtration. Man erhält 230 bis 270 g feuchten Filterkuchen entsprechend 185 bis 220 g Trockenware (ca. 83%ig an 4,4'-Dinitrostilben-2,2'-disulfonsäure). Nach Analyse mit Hilfe der HPLC-Methode und der Bestimmung des Kohlenstoffgehaltes in dem getrockneten Filterkuchen sowie der Mutterlauge ermittelte man eine Ausbeute von 94 % d.Th. an 4,4'-Dinitrostilben-2,2'-disulfonsäure.

Le A 23 691

Patentansprüche

1. Verfahren zur Herstellung von 4,4'-Dinitrostilben-2,2'-disulfonsäuresalzen durch Oxidation von 4-Nitrotoluol-2-sulfonsäure oder deren Salzen in wäßrig-alkalischem Medium, dadurch gekennzeichnet, daß man während der Reaktion Kalium-, Calcium- und/oder Magnesiumionen in dem Maße zugibt wie 4,4'-Dinitrostilben-2,2'-disulfonsäure gebildet wird, wobei zu jedem Zeitpunkt der Reaktion die Menge der zugegebenen Kalium-, Calcium- und/oder Magnesiumionen 10 bis 150 Mol-%, bezogen auf die jeweils im Reaktionsgemisch vorhandene Menge an 4,4'-Dinitrostilben-2,2'-disulfonsäure beträgt, und das ausgefallene Salz der 4,4'-Dinitrostilben-2,2'-disulfonsäure abtrennt.

2. Verfahren nach Anspruch 1, dadurch gekennzeichnet, daß zu jedem Zeitpunkt der Reaktion die Menge der zugegebenen Kalium-, Calcium- und/oder Magnesiumionen 20 bis 140 Mol-%, bezogen auf die jeweils im Reaktionsgemisch vorhandene Menge an 4,4'-Dinitro-stilben-2,2'-disulfonsäure beträgt.

3. Verfahren nach Ansprüchen 1 und 2, dadurch gekennzeichnet, daß zu jedem Zeitpunkt der Reaktion die Menge der zugegebenen Kalium-, Calcium- und/oder Magnesiumionen 50 bis 130 Mol-%, bezogen auf die jeweils im Reaktionsgemisch vorhandene Menge an 4,4'-Dinitrostilben-2,2'-disulfonsäure beträgt.

4. Verfahren nach Ansprüchen 1 bis 3, dadurch gekennzeichnet, daß man als Kalium-, Calcium- und/oder Magnesiumionen-liefernde Verbindungen die entsprechenden Hydroxide, Chloride und/oder Sulfate einsetzt.

5. Verfahren nach Ansprüchen 1 bis 4, dadurch gekennzeichnet, daß man die Umsetzung bei Temperaturen von 30 bis 100°C durchführt.

6. Verfahren nach Ansprüchen 1 bis 5, dadurch gekennzeichnet, daß man die 4,4'-Dinitrostilben-2,2'-disulfonsäure im wäßrig-alkalischen Medium in Mengen von 0,5 bis 30 g/l vorlegt, danach die 4-Nitrotoluol-2-sulfonsäure zugibt und während der Oxidationsreaktion Kalium-, Calcium- und/oder Magnesiumionen in dem Maße zugibt wie 4,4'-Dinitrostilben-2,2'-disulfonsäure gebildet wird.

Le A 23 691

# EUROPÄISCHER RECHERCHENBERICHT

Europäisches
Patentamt

**0 203 506**
Nummer der Anmeldung

EP 86 10 6873

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A,D | EP-A-0 026 154 (CIBA-GEIGY) | | C 07 C 143/55 |
| | --- | | |
| A,D | GB-A-2 136 430 (NIPPON KAYAKU) | | |
| | ----- | | |

| RECHERCHIERTE SACHGEBIETE (Int. Cl.4) |
|---|
| C 07 C 143/00 |

Der vorliegende Recherchenbericht wurde für alle Patentansprüche erstellt.

| Recherchenort DEN HAAG | Abschlußdatum der Recherche 28-08-1986 | Prüfer VAN GEYT J.J.A. |
|---|---|---|

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82